(19) Europäisches Patentamt
European Patent Office
Office européen des brevets

(11) Publication number: **0 180 372 B1**

(12) **EUROPEAN PATENT SPECIFICATION**

(45) Date of publication of patent specification: **08.01.92**

(51) Int. Cl.5: **C07D 501/36**

(21) Application number: **85307417.7**

(22) Date of filing: **15.10.85**

The file contains technical information submitted after the application was filed and not included in this specification

(54) **Crystalline cephalosporin antibiotics.**

(30) Priority: **25.10.84 US 664651**

(43) Date of publication of application:
**07.05.86 Bulletin 86/19**

(45) Publication of the grant of the patent:
**08.01.92 Bulletin 92/02**

(84) Designated Contracting States:
**BE CH DE FR GB IT LI NL SE**

(56) References cited:
**EP-A- 0 036 812**

(73) Proprietor: **THE UPJOHN COMPANY**
**301 Henrietta Street**
**Kalamazoo, Michigan 49001(US)**

(72) Inventor: **Amin, Mahendra Ishverbhai**
**c/o The Upjohn Company 301 Henrietta Street**
**Kalamazoo Michigan 49001(US)**
Inventor: **Campbell, Jay Allan**
**c/o The Upjohn Company 301 Henrietta Street**
**Kalamazoo Michigan 49001(US)**

(74) Representative: **Perry, Robert Edward et al**
**GILL JENNINGS & EVERY 53-64 Chancery Lane**
**London WC2A 1HN(GB)**

**EP 0 180 372 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to cephalosporin antibiotics in crystalline form.

US-A-4464367 discloses the compound of the formula

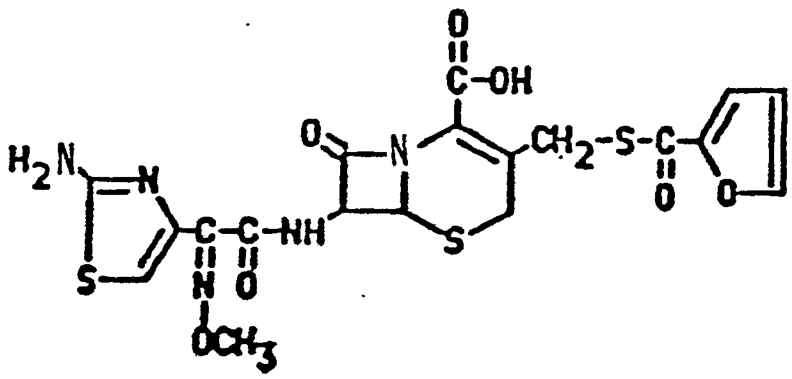

Formula (2)

i.e. 7-[2-(2-amino-1,3-thiazol-4-yl)-2-(methoxyimino)acetamido]-3-[(fur-2-ylcarbonyl)thiomethyl]-3-cephem-4-carboxylic acid or, by alternative nomenclature, 7-[2(2-amino-4-thiazolyl)-2-(methoxyimino)acetamido]-3-[(2-furanyl)carbonylthiomethyl]-8-oxo-5-thia-1-azabicyclo-[4.2.0]oct-2-ene-1-carboxylic acid, and its alkaline metal, alkaline earth metal and amine salts (of the carboxylic acid group), and easily-hydrolysable esters. The cephalosporin free acid of the given formula is now known by the generic name Ceftiofur, in France.

Ceftiofur and the derivatives disclosed in US-A-4464367 are somewhat unstable, chemically. They are obtained as amorphous compounds which are difficult to purify.

Novel compounds according to the present invention are Ceftiofur hydrochloride and Ceftiofur hydrobromide, preferably, in crystalline form. These crystalline salts are easier to isolate, purify and handle than Ceftiofur and its known derivatives. The novel compounds have the formula

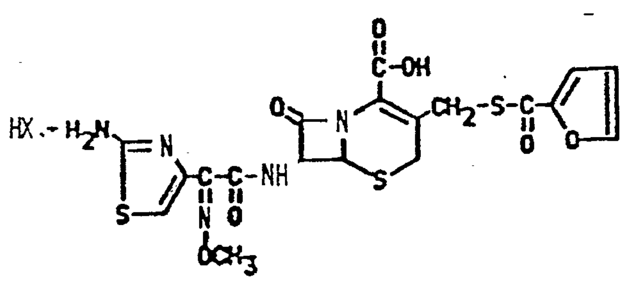

Formula (1)

wherein X is Cl or Br.

The novel salts may be prepared by the steps of

(a) heating a solution of 7-[2-(2-N-tritylamino-1,3-thiazol-4-yl)-2-(methoxyimino)acetamido]-3-(fur-2-ylcarbonyl)thiomethyl]-3-cephem-4-carboxylic acid in a polar organic solvent and water with an at least stoichiometric equivalent of hydrogen chloride or hydrogen bromide;

(b) reducing the concentration of the polar organic solvent in the aqueous phase, to form the crystalline hydrohalide salt;

(c) separating the crystalline hydrohalide salt from the slurry; and

(d) washing the separated salt with water and a polar organic solvent, and drying the salt.

In step (a), the N-trityl compound of the formula

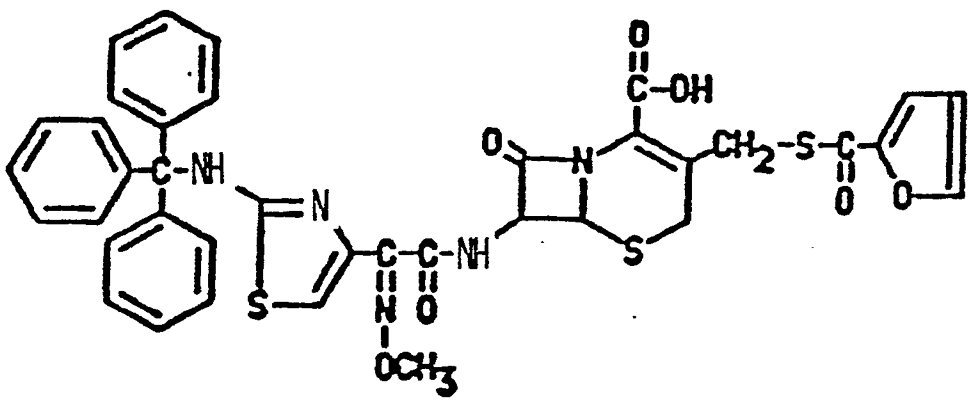

Formula (3)

is treated with a solution containing a water-miscible organic solvent such as tetrahydrofuran, acetonitrile, methyl ethyl ketone or, preferably, acetone, plus water and the hydrogen halide.

In step (b), a non-polar, water-immiscible organic liquid, e.g. toluene or heptane, may be added to reduce the concentration of the polar organic solvent, e.g. to extract acetone, and to take up the trityl alcohol by-product. Some of the water-miscible organic solvent may be separated by distillation or by other physical or chemical means. Water and hydrogen halide may be added to enhance crystal salt formation.

Alternatively, the novel salts can be prepared from Ceftiofur free acid or from an alkali metal, alkaline earth metal or amine salt thereof, e.g. the sodium carboxylate salt, by treatment in an aqueous/organic liquid mixture, e.g. of acetone and water, with the hydrogen halide. The desired salt is then precipitated, either by removing some of the organic liquid or by removing the aqueous phase from the mixture. The crystalline precipitate can be reoovered by known means.

As a matter of economics, the hydrochloride salt of the invention is generally preferred. The crystalline hydrochloride salt has the following X-ray powder diffraction pattern when crystallised from water-acetone:

| Interplanar d-spacings Å | intensity (relative %) |
|---|---|
| 18.4 | 44.2 |
| 12.4 | 73.1 |
| 8.26 | 50.0 |
| 7.82 | 100.0 |
| 7.69 | 17.9 |
| 6.19 | 48.1 |
| 5.86 | 32.1 |
| 5.21 | 23.1 |
| 5.12 | 40.4 |
| 4.74 | 30.1 |
| 4.37 | 21.8 |
| 4.23 | 13.5 |
| 3.98 | 26.9 |
| 3.91 | 35.9 |
| 3.81 | 17.9 |
| 3.30 | 14.1 |
| 3.01 | 12.8 |
| 2.88 | 14.1 |

(Peaks with relative intensity equal to or less than 10% are not reported.)

The salt may contain 0.5 to 2.0 equivalents of hydrogen chloride, but most often around one equivalent of HCl.

The crystalline hydrochloride salt can be crystallized in a solvent consisting of a polar organic solvent or water or a mixture of such solvents. A typical crystallization involves slurrying one gram free acid (2) in 20 ml. of 3% V/V water in acetone at 25° C. At least two equivalents of hydrogen chloride are added to dissolve the free acid and crystallize the hydrochloride salt. The crystals can be separated by filtration and washed to remove the remaining mother liquid. The crystals may then be dried under vacuum at elevated temperature. The yields of hydrochloride salt are generally 60% to 95% depending on the solvent and the

purity of the free acid (2).

This salt may contain 0.5% to 7% of water or other solvents (usually 1%-3%). Other impurities are usually reduced to 1% or less.

A recrystallization may be performed by either (a) forming and isolating the free acid (2) and then reforming the hydrochloride salt (1) or (b) by dissolving the hydrochloride salt (1) in aqueous organic solvent (usually containing one or more equivalents of HCl) and then removing the organic solvent by distillation, extraction, or other method of separation.

The free acid (2) or other pharmaceutically acceptable salts (such as (4)) can be made from the purified hydrochloride salt.

Other crystallization solvents include acetonitrile, tetrahydrofuran, methyl ethyl ketone and water. The hydrobromide salt can be made by a similar procedure.

The hydrochloride salt can also be made directly from the trityl-protected cephalosporin. For example, the crude trityl cephalosporin is slurried in 37% V/V water in acetone. Three equivalents of hydrogen chloride are added and the solution is refluxed until detritylation is complete. Toluene or equivalent non-polar water immiscible organic liquid solvent is added while stirring. The mixture is allowed to separate and the aqueous phase is removed and cooled to crystallize the hydrochloride salt. See Example 4 hereinbelow.

In aqueous buffer solutions the sodium salt (Formula 4) is more stable as the pH decreases. In hydrochloric acid at pH values equal to or lower than 2.8 the sodium salt (4) is converted to the hydrochloride salt (1) which exhibits better shelf stability when it is separated, washed and dried.

The new hydrochloride salt (1) has lower aqueous solubility than the sodium salt (4) and the free acid (2), and is better adapted for making controlled release pharmaceutical dosage formulations such as oral and parenteral suspensions, suppository and tablet dosage forms.

The new hydrochloride salt (1) produces acceptable pharmaceutical dispersions in water for injection and in pharmaceutical vegetable oils. Due to this property, the hydrochloride salt (1) can be formulated into extemporaneous (liquid and drug powder mixed just prior to administration) liquid suspensions by the addition of an aqueous or an oily vehicle to the hydrochloride salt (I) powder, just prior to dosing the patient. Oral and subcutaneous studies in mice with aqueous and oil dispersions of both the hydrochloride (1) and the sodium salt (4) indicate that the hydrochloride (1) is bioequivalent to the sodium salt (4). However, we have found that the crystalline hydrochloride salt (1) can be made in more pure form, is easier and is less expensive to manufacture than the sodium salt (4). Further, since the hydrochloride (1) has more limited solubility in water various controlled release pharmaceutical dosage formulations can be made with it (1) that are not readily adaptable to the sodium salt (4).

Due to the lower solubility of the ceftiofur hydrochloride, its rate of dissolution is slower. As a result absorption is less, and thus various controlled release formulation(s) can be formulated.

The controlled release parenteral suspension (see Examples 12 and 14 below) can be given once every 2 to 5 days as opposed to being given daily with other formulations.

The invention, how to make it and how to use it are further exemplified by the following detailed examples which are not intended to be limiting. In these examples, "povidone" is a known form of polyvinylpyrrolidone, used in pharmacy. "Quatresin" is a brand name for myristyl gamma-picolinium chloride. The corn oil glycerol monostearate gel and cottonseed oil glycerol monostearate gel materials are described in U.S. Patent No. 4,034,099. "Suppocire" refers to a brand name of a series of mixtures of saturated glycerides of $C_{10}$ to $C_{18}$-fatty acids which are known in pharmacy as excipients for use in various suppository and other pharmaceutical formulations and are described in product bulletins of Gatte fossé Establissment SA., Paris, France, 39 Avenue Edouard-Vaillant: "Suppocire AM" is said to have a melting point of 35° C. to 36.5° C. PEG-400 and PEG-8000 are well known pharmaceutical excipient forms based upon polyethylene glycol. Carnauba wax is the pharmaceutical form thereof defined in the National Formulary (NF).

The improved hydrohalide salt forming and purification process of this invention is adaptable to and can be used with a proposed large scale process for making the cephalosporin compound itself.

The N-trityl cephalosporin derivative (3) which is the preferred starting material is prepared by acylation of the corresponding 7-amino precursor 3-cephem-4-carboxylic acid nucleus compound with the N-trityl-2-amino-1,3-thiazol-4-yl-2-methoxyimino acetic acid by acylation methods well known to those skilled in the cephalosporin production art. For example, the procedure described in U.S. Patent 4,464,367 for the preparation of (3) with N-hydroxybenzotriazole and N,N-dicyclohexylcarbodiimide can be used. Alternately, the corresponding acid chloride can be prepared and used as described in the Journal of Antibiotics, 36, 180 (1983).

According to the process of this invention in step (a) the N-trityl-cephalosporin(3) (see STRUCTURE SHEET) is treated with a solution mixture of a polar solvent (e.g., acetone) and water (in proportions ranging

from about 200 to 100 parts V/V of polar solvent per 100 parts of water) in an amount to obtain a workable slurry. Sufficient hydrogen halide (e.g., hydrogen chloride) is then added. The hydrogen halide can be added in an amount which is at least stoichiometrically equivalent to the trityl-amino compound (3). For efficiency reasons, we prefer to add about three equivalents of hydrogen halide per equivalent of the trityl-protected oephalosporin (3). The hydrogen halide can be added as a gas, below the liquid surface, or as a hydrohalic acid solution in water.

In step (b), the acidified mixture from step (a) is heated to a temperature, generally above 45° C., and for a time sufficient to effect detritylation of the cephalosporin (3) and to form the cephalosporin hydrohalide salt (1). We have found that reflux of the mixture, which occurs in our mixtures at about 56° C., for about one hour is sufficient to effect the detritylation, without destruction of the cephalosporin.

In step (c) of the process the detritylated cephalosporin hydrohalide mixture is washed with a non-polar non-water miscible organic liquid which liquid takes up the trityl alcohol by-product and some of the acetone in the mixture. Examples of useful liquids for this purpose include liquid aromatic hydrocarbons such as benzene, toluene, xylene, chlorinated aromatic hydrocarbons such as chlorinated benzene, toluene and xylene, and chlorinated alkanes such as methylene chloride, dichloroethane, chloroform, carbon tetrachloride, as well as $C_2$ to $C_3$-alkyl acetate and propionate esters such as ethyl acetate, propyl acetate and liquid $C_6$ to $C_{10}$-alkanes such as hexanes, heptane, octane, nonane and decane, and liquid $C_5$ to $C_7$-cycloalkanes such as cyclopentane, cyclohexane, cycloheptane, and mixtures, including commercial mixtures of such liquids. Toluene and/or heptane are preferred. Optionally, extra water and acid may also be added at this point. The hydrohalic acid addition is optional because it depends upon the amount of hydrogen halide already present in the mixture. The added hydrogen halide at this stage gives shorter cyrstallization times.

In step (d) the crystalline cephalosporin hydrohalide salt (1) is separated from the liquid phase by known means such as by filtration or centrifugation procedures, and in step (e) the separated crystalline cephalosporin hydrohalide salt (1) is washed one or more times with water and water-miscible organic solvent, e.g., acetone, or mixtures thereof, and then dried (step f), for example, by spreading the crystalline salt in trays, and drying the salt in a vacuum oven at 40°-60° C. for a time sufficient to remove any adhering volatile liquids therefrom.

The compounds of Formula 1 herein are useful as the active antibiotic drug compound in pharmaceutical dosage forms for treating valuable mammalian animals and humans to treat bacterial infections in that valuable animal or human. Presently it is contemplated that this compound will be especially useful as a veterinary antibiotic drug to treat valuable animals such as cattle, horses, goats, dogs and cats to fight the effects of bacterial infections caused by organisms such as Pasturella hemolitica, Salmonella typhimurium, E. coli Staphylococcus, aureus, and the like, some of which are commonly associated with 'shipping fever' in animals.

The term "dosage unit form" as used in this specification and in the claims refers to physically discrete units suitable as unitary dosages for mammalian subjects, each unit containing as the essential active ingredient a predetermined quantity of a compound of this invention with the required pharmaceutical means which adapt said ingredient for systemic administration. The specification for the novel dosage unit forms of this invention are dictated by and directly dependent on the physical characteristics of the essential active ingredient and the particular effect to be achieved in view of the limitations inherent in the art of compounding such an essential active material for beneficial effects in humans and animals as disclosed in detail in this specification. Examples of suitable dosage unit forms in accordance with this invention are tablets, capsules, orally administered liquid preparations in suitable liquid vehicles, sterile preparations in suitable liquid vehicles for intramuscular and intravenous administration, suppositories and sterile dry preparations for the extemporaneous preparation (mixing just prior to administration) of sterile injectable preparations in a suitable liquid vehicle. Suitable solid diluents or carriers for the solid oral pharmaceutical dosage unit forms are selected from the group consisting of lipids, carbohydrates, proteins and mineral solids, for example, starch, sucrose, lactose, kaolin, dicalcium phosphate, gelatin, acacia, corn syrup, corn starch, talc and the like. Capsules, both hard and soft, are filled with compositions of this antibiotic active ingredient in combination with suitable diluents and excipients, for example, edible oils, talc, calcium carbonate and the like and also calcium stearate. Liquid preparations for oral administration are prepared in water or aqueous vehicles which advantageously contain suspending agents, for example, methylcellulose, alginates, tragacanth, pectin, kelgin, carragenan, acacia, polyvinylpyrrolidone, polyvinyl alcohol, and the like, to increase the viscosity of the composition. In the case of injectable forms, the injectable formulation must be sterile and must be fluid to the extent that easy syringeability exists. Such preparations must be stable under the conditions of manufacture and storage, and ordinarily contain in addition to the principal solvent or suspending liquid, preservatives in the nature of bacteriostatic and

fungistatic agents, for example, parabens, chlorobutanol, benzyl alcohol, benzoic acid, phenol, thimerosal, and the like to preserve the composition against microorganisms. In many cases, it is preferable to include osmotically active agents, for example, sugars or sodium chloride in isotonic concentrations. Carriers and vehicles include vegetable oils, dimethylacetamide, dimethylformamaide, ethyl lactate, ethyl carbonate, isopropyl myristate, ethanol, polyols, for example, glycerol, propylene glycol, liquid polyethylene glycol, and the like. Any solid preparations for subsequent extemporaneous preparation of sterile injectable preparations are sterilized, by exposure to steam, cobalt 60 irradiation, or by exposure to a sterilizing gas, for example, ethylene oxide. The aforesaid carriers, vehicles, diluents, surfactants, excipients, preservatives, isotonic agents and the like constitute the pharmaceutical means which adapt the preparations for systemic administration.

In these pharmaceutical compositions it may be desirable to include a viscosity increasing agent such as sodium carboxymethylcellulose (sodium CMC). Other suitable viscosity increasing agents can be substituted for sodium CMC.

The pharmaceutical dosage unit forms of the compounds of this invention are prepared in accordance with the preceding general description to provide from about 1 mg. to about 500 mg. of the essential active ingredient per dosage unit form, which as aforesaid may be in the form of a semi-solid or solid, topical, oral or rectal preparation, a liquid oral preparation, an injectable preparation including liquid preparations and solid dry preparations for extemporaneous reconstitution to a liquid injectable preparation. The amount of the essential active ingredient provided in the pharmaceutical dosage unit forms is that amount sufficient to obtain antibiotic effects within the aforesaid effective non-toxic range. Expressed otherwise, when used systemically, an amount of the essential active ingredient is provided to a recipient within a range from about 0.2 mg./kg. to about 10 mg./kg. of body weight of the recipient.

Preferred dosages for most applications are 0.2 mg./kg. to 5.0 mg./kg. of body weight. In a topical semi-solid ointment formulation the concentration of the active ingredient may be 1%-20%, preferably 5%-10% in a carrier, such as a pharmaceutical cream base.

The useful pharmaceutical dosage unit forms of these compounds in pharmaceutical formulations are preferably adapted for systemic administration to obtain antibiotic effects comprising an effective, non-toxic amount of the Formula 1 salt.

Further, the invention relates to methods of obtaining antibiotic effects in mammals, for example, humans and valuable warm-blooded animals such as dogs, cats, horses, and other commercially valuable animals, by administering systemically to the mammals the aforesaid pharmaceutical dosage units forms supplying an effective, non-toxic amount for antibiotic effects.

The invention is further illustrated by the following detailed examples.

Example 1

A slurry of 0.7 g. of ceftiofur as its free acid in 7 ml. of acetone and 0.35 ml. of water is made up. To this slurry is added 0.24 ml. of 37.7% hydrochloric acid solution. The resulting acidified slurry becomes a solution within a minute, and then crystallization of the ceftiofur hydrochloride salt begins within 2 or 3 minutes. An additional 7 ml. of acetone are added to thin the slurry. The slurry mixture is stirred for 10 minutes at 20° C. and then filtered and the filtered hydrochloride salt crystals are washed with acetone. The ceftiofur hydrochloride salt crystals are then dried for 16 hours in a vacuum oven. The yield of the ceftiofur hydrochloride salt crystals is 0.63 g., for an 84% chemical yield.

Example 2

A slurry of 0.7 g. of ceftiofur free acid in 11.8 ml. of acetone and 6.9 ml. of water is made up. To this slurry there is added 0.27 ml. of 37.7% hydrochloric acid solution, and the mixture is warmed to 55° C. The ceftiofur free acid is in solution. To this warmed solution a 55° C. temperature mixture of 13.2 ml. of toluene and 3.2 ml. of water is added while stirring. The liquid phases are allowed to separate, and the upper organic layer is discarded. The lower aqueous layer is cooled to 15° C. after seeding with previous crystalline ceftiofur hydrochloride, and allowed to stand for two hours. After standing, the mixture is filtered to separate the crystalline ceftiofur hydrochloride salt from the liquid mother liquor. The filtered salt crystals are washed with 4 ml. of 20% acetone in water V/V mixture. Then the salt crystals are washed with 10 ml. of water. The washed ceftiofur hydrochloride salt crystals are dried for 16 hours in a vacuum oven at 40° C. The yield of ceftiofur hydrochloride salt crystals is 0.50 g. for a 67% chemical yield.

Example 3 Detritylation of N-trityl-ceftiofur and Crystallization of Ceftiofur Hydrochloride Salt Using Toluene

Extraction of Acetone

To a three-necked, round-bottom 250 ml. flask is charged 14.8 g. of N-tritylated ceftiofur free acid (3), 91 ml. of acetone, 2.4 ml. of concentrated hydrochloric acid and 91 ml. of water. This resulting mixture is heated to reflux. Then 10 ml. more of acetone is added to dissolve a precipitate, presumably trityl alcohol.

While the mixture is still hot (55° C.), 60 ml. of toluene is added to remove trityl alcohol and to decrease the concentration of acetone in the aqueous phase. The resulting mixture is transferred to a separatory funnel and the liquid layers are separated. The organic liquid phase is washed with a mixture of 25 ml. of water and 10 ml. of acetone. The organic layer is 120 ml. total volume. The separated aqueous layer is washed with 30 ml. of toluene, and then the toluene and aqueous phases are separated to give a 45 ml. total volume toluene plus inter-phase. The aqueous layer (about 140 to 150 ml.) is stirred with a magnetic stir bar for three hours, and then cooled to -5° C. and filtered to separate the ceftiofur hydrochloride salt crystals which form. This crystalline filtered salt product is washed with 15 ml. of a mixture consisting of 10 ml. of water and 5 ml. of acetone. The washed ceftiofur hydrochloride crystalline product, 4.63 g., is dried at 50° C.

A second crop can be obtained by filtering mother liquor filtrate. The resulting ceftiofur hydrochloride crystalline product, 1.46 g., is washed with water. The total yield is 88.1%.

Example 4

A mixture of 96 ml. acetone, 55 ml. water and 2.93 ml. 37.7% HCl in water solution are added to about 10.0 gm. trityl cephalosporin (3) and refluxed (about 58° C.) for one hour to affect detritylation.

A mixture of 18 ml. heptane and 4.5 ml. acetone is added to the above solution. The combined liquids are transferred to a separatory funnel and allowed to separate. The lower layer (aqueous) is transferred to another separatory funnel and 18 ml. of heptane and 4.5 ml. of acetone are added to the aqueous phase in the second separatory funnel. The combined liquids are contacted and allowed to separate as before. The lower phase (aqueous) is distilled atmospherically to a temperature of 67° C. which reduces the concentration of acetone to about 15%. Before cocling to 15° C., 2.93 ml. of 37.7% HCl solution are added. The cephalosporin HCl salt (1) begins crystallizing between 50° C. and 60° C.

The crystals are filtered and washed with 20 ml. of 10% acetone in water to remove any adhering filtrate. The resulting washed, filtered solids are then washed with water and dried in a vacuum oven at 65° C. to give 6.87 g. of product.

Example 5 Oral Suspension

One thousand cc. of an aqueous suspension for oral use, containing in each 5 cc. dose 5 to 300 mg. of crystalline ceftiofur hydrochloride is prepared from the following types of and amounts of ingredients.

| | |
|---|---|
| Ceftiofur hydrochloride (crystalline) | 5 to 300 gms. |
| Benzoic Acid or Sorbic Acid | 1 gm. |
| Sucrose | 650 gms. |
| Sodium Carboxymethylcellulose, Low Viscosity | 1 to 20 gms. |
| Flavors (e.g., USP cherry, orange) q.s. | |
| Sodium Chloride (0.5 to 10 mg./ml.) | 0.5 to 10 gms. |
| Hydrochloric Acid, Reagent Grade | q.s. adjust pH to approx. 3.0 |
| Deionized Water | q.s. to 1000 cc. |

The sodium carboxymethylcellulose, benzoic acid, sucrose, appropriate flavors and sodium chloride are dispersed in sufficient water to make 650 mls. of solution. The ceftiofur hydrochloride is stirred into the syrup until uniformly distributed. The resulting suspension is colloid milled to a uniform consistency. Sufficient water is added to bring volume to 900 cc. If necessary pH is adjusted with hydrochloric acid to

about pH3. Sufficient water is added to make 1000 cc.

Example 6 Sterile Parenteral Suspension

| | | |
|---|---|---|
| **Sterile Vehicle - Part I** | | |
| PEG | | 5 to 120 gms. |
| Benzyl Alcohol, or | | 9.1 gm. |
| Benzoic Acid | | 1.0 gm. |
| Povidone | | 1 to 10 gms. |
| Sodium Chloride Fine Crystals, Reagent Grade | | 9 gms. |
| Hydrochloric Acid, Reagent Grade | q.s. adjust pH to approximately 3.0 | |
| 50% Solution Sodium Hydroxide | q.s adjust pH | |
| Water for Injection | q.s adjust | 1000 cc. |
| **Part II** | | |
| Ceftiofur hydrochloride, crystalline | | 1.0 to 100 gms. |
| Vehicle Part I | q.s adjust | 1000 cc. |

DIRECTIONS

Part I

All of the ingredients are dissolved in water and pH adjusted to about 2.6 to 3.2, preferably about 3.0. The vehicle sterilized by filtration and used in Part II.

Part II Aseptically add sterile crystalline ceftiofur hydrochloride in sufficient vehicle from Part I to make 900 mls. Stir the suspension and colloid mill the suspension to a uniform consistency. Add sufficient vehicle to make 1000 mls.

Example 7 Sterile Parenteral Suspension

| | |
|---|---|
| **Sterile Vehicle - Part I** | |
| Polysorbate 80, N.F. | 0.1 to 10 gms. |

| | | |
|---|---|---|
| Sodium Carboxymethylcellulose low viscosity | | 2 to 20 gms. |
| Benzyl Alcohol | | 9.1 gms. |
| Benzoic Acid | | 0.2 to 2.0 gms. |
| Povidone | | 1 to 10 gms. |
| Sodium Chloride, Fine Crystals Reagent if needed | | 9 gms. |
| Hydrochloric Acid, Reagent Grade | q.s. adjust pH to approx. 3.0 | |
| 50% Solution Sodium Hydroxide | q.s. adjust pH | |
| Water for Injection | q.s. adjust | 1000 cc. |
| Part II | | |
| Ceftiofur hydrochloride, crystalline | | 1.0 to 100 gm.s |
| Vehicle Part I | q.s. adjust | 1000 cc. |

Directions:

Part I

All of the ingredients are dissolved in water and the vehicle sterilized by filtration.

Part II

Aseptically add sterile crystalline ceftiofur in sufficient vehicle tc make 900 mls. Stir the suspension and pass through colloid mill to a uniform consistency. Add sufficient vehicle to make 1000 mls.

Example 8 Sterile Parenteral Suspension

| | | |
|---|---|---|
| Sterile Vehicle - Part I | | |
| PEG 3350 NF | | 5 to 120 gms. |
| Benzyl Alcohol | | 9.1 gms. |
| Benzoic Acid | | 0.2 to 2.0 gms. |
| Polysorbate 80 NF Food Grade | | 1 to 5 gms. |
| Sodium Chloride Fine Crystals Reagent | | 0.5 to 10 gms. |
| Hydrochloric Acid, Reagent Grade | q.s. adjust pH to appox. 3.0 | |
| 50% Solution Sodium Hydroxide | q.s. adjust pH | |
| Water for Injection | q.s. adjust | 1000 cc. |
| Part II | | |
| Ceftiofur hydrochloride, crystalline | | 1 to 100 gms. |
| Vehicle Part I | q.s. adjust | 1000 cc. |

Directions:

Part I

All of the ingredients are dissolved in water and pH adjusted to approximately 3.0, and the vehicle sterilized by filtration.

Part II

Aseptically add sterile crystalline ceftiofur hydrochloride in sufficient vehicle from Part I to make 900 mls. Stir the suspension and pass through a colloid mill to a uniform consistency. Add sufficient vehicle to make 1000 mls.

Example 9 Sterile Extemporaneous Parenteral Suspension (Aqueous)

| | |
|---|---|
| Sterile Vehicle - Part I | |
| Benzyl Alcohol or | 9.1 gms. or |
| Benzoic Acid | 0.2 to 2.0 gms. |
| Carboxymethylcellulose Sodium USP low viscosity or any other viscosity inducing agent | 1.0 to 20.0 gms. |
| Sodium Chloride Fine Crystals, Reagent Grade | 0.5 to 10 gms. |
| Hydrochloric Acid, Reagent Grade | q.s. adjust pH to approx. 3.0 |
| Water for Injection | |
| Part II | Amount per Vial |
| Sterile Crystalline ceftiofur, hydrochloride in a 10 to 100 ml. glass vial | 0.01 to 1.5 gm. |

Directions:

Part I

All of the ingredients are dissolved in water, and pH adjusted to approximately 2.6 to 3.2, preferably about 3.0. Vehicle sterilized by filtration and packaged in appropriate glass vials.

Part II

Sterile crystalline ceftiofur hydrochloride is packaged aseptically in sterile vials or crystalline ceftiofur hydrochloride is first packaged and the final container(s) sterilized by Cobalt 60 irradiation.

Example 10 Sterile Extemporaneous Parenteral Suspension

| | | |
|---|---|---|
| Sterile Vehicle Part I | | |
| Methylparaben | | 1.0 to 2.7 gms. |
| Propylparaben | | 0.1 to 0.5 gm. |
| Povidone | | 1 to 10 gms. |
| Sodium Chloride Fine Crystals Reagent Grade | | 0.5 to 10 gms. |
| 20% Solution Hydrochloric acid | q.s. adjust pH to approx. 3.0 | |
| 50% Solution Sodium Hydroxide | q.s. adjust pH | |
| Water for Injection | q.s. adjust | 1000 ccs. |

| Part II | Amount Per Vial |
| --- | --- |
| Sterile crystalline ceftiofur hydrochloride in a 10 to 100 ml. glass vial | 0.01 to 1.5 gm. |

Directions

Part I

Methylparaben and propylparaben are dissolved in boiling water. Then all of the ingredients dissolved in water, and pH adjusted to approximately 2.6 to 3.2, preferably about 3.0. Vehicle sterilized by filtration and packaged in appropriate glass vials.

Part II

Sterile crystalline ceftiofur hydrochloride is packaged aseptically in sterile vials or crystalline ceftiofur hydrochloride is first packaged and the final container(s) shall be sterilized by Cobalt 60 irradiation.

Example 11 Extemporaneous Parenteral Suspension (Aqueous)

| Sterile Vehicle - Part I | | |
| --- | --- | --- |
| Polyethylene Glycol 3350 NF | | 5 to 120 gms. |
| Polyvinyl Pyrrolidone | | 1 to 10 gms. |
| Quatresin® myristyl gamma picolinium chloride | | 0.1 to 2.0 gms. |
| Sodium Chloride, Fine Crystals Reagent Grade | | 0.5 to 10 gms. |
| 20% Solution Hydrochloric Acid | q.s. adjust pH to approx. 3.0 | |
| 50% Solution Sodium Hydroxide | q.s. to adjust pH to approx. 3.0 | |
| Water for Injection | q.s. adjust to | 1000 cc. |

| Part II | Amount Per Vial |
| --- | --- |
| Sterile crystalline ceftiofur hydrochloride (milled or micronized) in a 10 to 100 ml. glass vial | 0.01 to 1.5 gms. |

DIRECTIONS

Part I

All of the ingredients are dissolved in water, and pH adjusted to approximately 2.6 to 3.2, preferably about 3.0. Vehicle sterilized by filtration and packaged in appropriate glass vials.

Part II

Sterile crystalline ceftiofur hydrochloride is packaged aseptically in sterile vials or crystalline ceftiofur hydrochloride is first packaged and the final container(s) are sterilized by Cobalt 60 irradiation.

Example 12 - Sterile Nonaqueous Parenteral Suspension

| | |
|---|---|
| Crystalline ceftiofur hydrochloride | |
| (milled or micronized) | 1 to 100 gms. |
| Chlorobutanol Anhydrous - preservative | 5.25 gms. |
| or | |
| Benzyl Alcohol | 9.25 gms. |
| Corn Oil Glyceryl Monostearate Gel | |
| or | |
| Cottonseed Oil Glyceryl Monostearate Gel | q.s. adjust |

DIRECTIONS

Preservative is dissolved in sufficient oily gel to make 800 cc. Crystalline ceftiofur hydrochloride is added, and the suspension is colloid milled to a uniform consistency. Add sufficient gel to make 1000 mls. After packaging into glass vials, the suspension is sterilized by Cobalt 60 irradiation or by any other suitable method.

Example 13 Sterile Nonaqueous Parenteral Suspension

| | | |
|---|---|---|
| Crystalline ceftiofur hydrochloride | | |
| (milled or micronized) | | 1 to 100 gms. |
| Chlorobutanol Anhydrous | | 5.25 gms. |
| or | | |
| Benzyl Alcohol | | 9.25 gms. |
| Corn Oil USP | q.s. adjust | 1000 cc. |
| or | | |
| Cottonseed oil | q.s. adjust | 1000 cc. |

DIRECTIONS

Preservative is dissolved in sufficient oil to make 800 cc. Crystalline ceftiofur hydrochloride is added, and the suspension is colloid milled to a uniform consistency to break the aggregates. Add sufficient amount of oil to make 1000 mls. Stir and package into glass vials. The suspension can be sterilized by Cobalt 60 irradiation or sterile crystalline ceftiofur hydrochloride can be added to sterile vehicle and manufactured following aseptic procedure(s).

Example 14 Sterile Extemporaneous Parenteral Suspension (Nonaqueous Gel) - Controlled Release Formulation

| Sterile Vehicle Part I | 1000 |
|---|---|
| Benzyl Alcohol - preservative | 9.0 to 9.25 gms. |
| or | |

| | |
|---|---|
| Chlorobutanol | 5.0 to 5.25 gms. |
| Corn Oil Glyceryl Monostearate Gel | 1000 cc. |
| or | |
| Cottonseed Oil Glyceryl Monostearate Gel | 1000 cc. |
| Part II | 100 Vials |
| Crystalline ceftiofur hydrochloride (milled or micronized) | 1 to 100 gms. |

DIRECTIONS

Part I

Preservative is dissolved in sufficient gel, and the gel is filled into vials asceptically and the vials sealed. These vials will be packaged with the vials of Part II as companion package.

Part II

0.01 to 1.0 gm. of crystalline ceftiofur hydrochloride or sterilized crystalline ceftiofur hydrochloride is packaged in a sterile glass vial and the vials sealed. If the crystalline ceftiofur hydrochloride is non-sterile, then the packaged vials will be sterilized by Cobalt 60 irradiation.

Prior to dosing appropriate amounts of Part I diluent will be added to Part II sterile powder and shaken until homogeneous.

Example 15 Sterile Extemporaneous Parenteral Suspension (Nonaqueous)

| Sterile Vehicle Part I | 1000 |
|---|---|
| Benzyl Alcohol - preservative | 9.0 to 9.25 gms. |
| or | |
| Chlorobutanol | 5.0 to 5.25 gms. |
| Corn Oil, USP q.s. ad. | 1000 cc. |
| or | |
| Cottonseed Oil, USP q.s. ad. | 1000 cc. |
| Part II | 100 Vials |
| Crystalline ceftiofur hydrochloride, (milled and micronized) | 50 to 100 gms. |

Part I

Preservative is dissolved in the oil, and the solution sterilized by filtration. The sterile solution is filled into vials and the vials sealed. These vials will be packaged with the vials of Part II as companion package.

Part II

0.5 to 1.0 gm. of crystalline ceftiofur hydrochloride or sterilized crystalline ceftiofur hydrochloride is packaged in a sterile glass vial and the vials sealed. If the crystalline ceftiofur hydrochloride is non-sterile, then the packaged vials will be sterilized by Cobalt 60 irradiation.

Prior to dosing appropriate amounts of Part I diluent will be added to Part II sterile powder and shaken until uniformly mixed.

Example 16 Suppositories

Formulation for a 2 gm. suppository containing 62.5 mg. of crystalline ceftiofur hycrochloride is given. However, any size suppository can be manufactured using any amount of ceftiofur hydrochloride and appropriate amounts of excipients at the same ratio as indicated below.

| | Lot Size 12 |
|---|---|
| Crystalline ceftiofur hydrochloride (milled or micronized) | 0.750 gm. |
| PEG-400 | 14.4 ml. |
| PEG-8000 | 9.6 gm. |

Directions

Measure out 14.4 ml. of PEG-400 and place in a container suitable for heating. Add 9.6 gms. of PEG-8000 (melting point 140° F.) to the PEG-400 solution and melt over a hot water bath approximately two minutes or until there is a clear solution.

Add crystalline ceftiofur hydrochloride and stir until dispersed. Pour the mix into the mold and let set. Chill the mold. Remove suppositories after they set up 15-30 minutes at room temperature. Sterile suppositories can be manufactured with sterile raw materials and observing aseptic conditions during manufacturing, or can be sterilized by Cobalt 60 irradiation.

Example 17 Suppositories

Suppositories can also be manufactured from excipients such as cocoa butter, SuppocireTM AM, Suppocire $AS_2$, and Suppocire AT, Suppocire BT or Suppocire CT brand of $C_8$ to $C_{10}$-saturated fatty acid glycerides.

Formula for a 2 gm. suppository containing 62.5 mg. of crystalline ceftiofur hydrochloride is given; however, any size suppository can be manufactured using any desired amount of crystalline ceftiofur hydrochloride, and appropriate amount of excipient.

| | Lot Size 12 |
|---|---|
| Crystalline ceftiofur hydrochloride (milled or micronized) Sterile | 0.750 gm. |
| Suppocire AM or $AS_2$, or AT, or BT or CT | 23.25 gm. |

Directions

Weigh the SuppocireTM diluent in a container suitable for heating. Melt (45° C. temperature) over a hot water bath for approximately two minutes or until there is a clear solution (microwave oven can also be used instead of the water bath). Sterilize by filtration. Add sterile crystalline ceftiofur hydrochloride and stir until dispersed. Pour the mix into the cold mold. After two to four minutes, the surplus of the casting is eliminated by scraping. The temperature and time of cooling must be governed according to the type of formula. The circulating cold air should come in contact with all faces of the mold. Release from the mold must be gentle. Sterile suppositories can be manufactured with sterile raw materials and observing aseptic conditions during manufacturing, or can be sterilized by Cobalt 60 irradiation.

Example 18 Capsules

One thousand two-piece hard gelatin capsules for oral use, each containing 50 mgs. of activity of crystalline ceftiofur hydrochloride are prepared from the following types and amounts of materials:

| | 1000 |
|---|---|
| Crystalline ceftiofur hydrochloride | (50 gms.) |
| or | |
| * Coated with Carnauba Wax® | |
| or | |
| * White Wax | |
| Talc and/or | 75 gms. |
| Magnesium Stearate | 25 gms. |

( ) = Activity of ceftiofur hydrochloride

* Coated crystalline ceftiofur hydrochloride will have controlled release properties. The materials are thoroughly mixed and then encapsulated in the usual manner. Different strength capsules can be prepared by changing the amounts of crystalline ceftiofur hydrochloride.

Example 19 Tablets

One thousand compressed tablets for oral use, each containing an amount equivalent to 50 mgs. crystalline ceftiofur hydrochloride can be prepared using the following:

| | |
|---|---|
| Ceftiofur hydrochloride crystalline | 50 gms. |
| Lactose | 375 gms. |
| Corn Starch | 65 gms. |
| Magnesium Stearate | 10 gms. |

The ingredients are thoroughly mixed and slugged. The slugs are broken down by forcing through a screen. The resulting mixture is then compressed into tablets. Different strength tablets can be prepared by appropriate changes in the amounts of ceftiofur hydrochloride and the excipients.

## Claims

1. Ceftiofur hydrochloride.

2. Ceftiofur hydrobromide.

3. A salt according to claim 1, in crystalline form.

4. A salt according to claim 2, in crystalline form.

5. A salt according to claim 3, which has the following X-ray powder diffraction pattern when crystallised from an acetone/water mixture;

| Interplanar d-spacings Å | intensity (relative %) |
|---|---|
| 18.4 | 44.2 |
| 12.4 | 73.1 |
| 8.26 | 50.0 |
| 7.82 | 100.0 |
| 7.69 | 17.9 |
| 6.19 | 48.1 |
| 5.86 | 32.1 |
| 5.21 | 23.1 |
| 5.12 | 40.4 |
| 4.74 | 30.1 |
| 4.37 | 21.8 |
| 4.23 | 13.5 |
| 3.98 | 26.9 |
| 3.91 | 35.9 |
| 3.81 | 17.9 |
| 3.30 | 14.1 |

**6.** A process for preparing a crystalline hydrohalide salt according to any of claims 3 to 5, which comprises the steps of

(a) heating a solution of 7-[2-(2-N-tritylamino-1,3-thiazol-4-yl)-2-(methoxyimino)acetamido]-3-(fur-2-ylcarbonyl)thiomethyl]-3-cephem-4-carboxylic acid in a polar organic solvent and water with an at least stiochiometric equivalent of hydrogen chloride or hydrogen bromide;

(b) reducing the concentration of the polar organic solvent in the aqueous phase, to form the crystalline hydrohalide salt;

(c) separating the crystalline hydrohalide salt from the slurry; and

(d) washing the separated salt with water and a polar organic solvent, and drying the salt.

**7.** A process according to claim 6, wherein, in step (c), a non-polar, water-immiscible organic liquid is added.

**8.** A process according to claim 7, wherein the water-immiscible organic liquid is toluene.

**9.** A process according to claim 7, wherein the water-immiscible organic liquid is heptane, and the mixture is distilled to remove polar organic liquid therefrom.

**10.** A salt according to any of claims 1 to 5, for alleviating the effects of undesired bacterial infections in warm-blooded mammals.

**Revendications**

**1.** Chlorhydrate de Ceftiofur.

**2.** Bromhydrate de Ceftiofur.

**3.** Sel suivant la revendication 1, sous forme cristalline.

4. Sel suivant la revendication 2, sous forme cristalline.

5. Sel suivant la revendication 3, qui possède le diagramme suivant de diffraction des rayons X sur poudre, lors de sa cristallisation dans un mélange acétone/eau :

| Distances d entre plans Å | Intensité (relative, %) |
|---|---|
| 18,4 | 44,2 |
| 12,4 | 73,1 |
| 8,26 | 50,0 |
| 7,82 | 100,0 |
| 7,69 | 17,9 |
| 6,19 | 48,1 |
| 5,86 | 32,1 |
| 5,21 | 23,1 |
| 5,12 | 40,4 |
| 4,74 | 30,1 |
| 4,37 | 21,8 |
| 4,23 | 13,5 |
| 3,98 | 26,9 |
| 3,91 | 35,9 |
| 3,81 | 17,9 |
| 3,30 | 14,1 |

6. Procédé de préparation d'un halogénhydrate cristallin suivant l'une quelconque des revendications 3 à 5, qui comprend les étapes consistant
(a) à chauffer une solution d'acide 7-[2-(2-N-tritylamino-1,3-thiazole-4-yl)-2-(méthoxyimino)-acétamido]-3-(fur-2-ylcarbonyl)thiométhyl]-3-céphem-4-carboxylique dans un solvant organique polaire et de l'eau avec au moins un équivalent stoechiométrique de chlorure d'hydrogène ou de bromure d'hydrogène ;
(b) à réduire la concentration du solvant organique polaire dans la phase aqueuse pour former l'halogénhydrate cristallin ;
(c) à séparer l'halogénhydrate cristallin de la suspension ; et
(d) à laver le sel séparé avec de l'eau et un solvant organique polaire, et à sécher le sel.

7. Procédé suivant la revendication 6, dans lequel, dans l'étape (c), un liquide organique non polaire, non miscible à l'eau, est ajouté.

8. Procédé suivant la revendication 7, dans lequel le liquide organique non miscible à l'eau est le toluène.

9. Procédé suivant la revendication 7, dans lequel le liquide organique non miscible à l'eau est l'heptane et le mélange est distillé pour en éliminer le liquide organique polaire.

10. Sel suivant l'une quelconque des revendications 1 à 5, destiné à réduire les effets d'infections bactériennes indésirables chez des animaux à sang chaud.

**Patentansprüche**

1. Ceftiofurhydrochlorid.

2. Ceftiofurhydrobromid.

3. Salz nach Anspruch 1 in Kristallform.

4. Salz nach Anspruch 2 in Kristallform.

5. Salz nach Anspruch 3, welches das folgende Pulverbeugungsdiagramm hat, wenn es von einer Aceton/Wassermischung kristallisiert wird;

| Netzebenenabstand d<br>A | Intensität<br>(Relativ%) |
|---|---|
| 18,4 | 44,2 |
| 12,4 | 73,1 |
| 8,26 | 50,0 |
| 7,82 | 100,0 |
| 7,69 | 17,9 |
| 6,19 | 48,1 |
| 5,86 | 32,1 |
| 5,21 | 23,1 |
| 5,12 | 40,4 |
| 4,74 | 30,1 |
| 4,37 | 21,8 |
| 4,23 | 13,5 |
| 3,98 | 26,9 |
| 3,91 | 35,9 |
| 3,81 | 17,9 |
| 3,30 | 14,1 |

6. Verfahren zur Herstellung eines kristallinen Hydrohalogenidsalzes nach einem der Ansprüche 3 bis 5, welches die folgenden Schritte umfasst:
   (a) Erhitzen einer Lösung von 7-[2-(2-N-Tritylamino-1,3-thiazol-4-yl)-2-(methoxyimino)acetamido]-3-(fur-2-ylcarbonyl)thiomethyl]-3-cephem-4-carbonsäure in einem polaren organischen Lösungsmittel und Wasser mit einem wenigstens stöchiometrischen Äquivalent von Hydrogenchlorid oder Hydrogenbromid;
   (b) Reduzieren der Konzentration des polaren organischen Lösungsmittels in der wässrigen Phase, um das kristalline Hydrohalogenidsalz zu bilden;
   (c) Trennen des kristallinen Hydrohalogenidsalzes von der Aufschlämmung; und
   (d) Waschen des getrennten Salzes mit Wasser und einem polaren organischen Lösungsmittels, und Trocknen des Salzes.

7. Verfahren nach Anspruch 6, worin eine nicht-polare, mit Wasser unvermischbare organische Flüssigkeit in Schritt (c) zugesetzt wird.

8. Verfahren nach Anspruch 7, worin die mit Wasser unvermischbare organische Flüssigkeit Toluol ist.

9. Verfahren nach Anspruch 7, worin die mit Wasser unvermischbare organische Flüssigkeit Heptan ist, und die Mischung destilliert wird, um polare organische Flüssigkeit davon zu entfernen.

10. Salz nach einem der Ansprüche 1 bis 5 zum Lindern der Einwirkungen von unerwünschten bakteriellen Infektionen in warmblütigen Säugetieren.